# EUROPEAN PATENT SPECIFICATION

(11) **EP 2 292 782 B1**
(45) Date of publication and mention of the grant of the patent: **17.01.2018**
(21) Application number: 08800799.2
(22) Date of filing: 08.09.2008
(51) Int. Cl.: C12P 7/64, C12N 1/12, C11B 1/10, C11C 3/00, C10L 1/02, C10G 3/00, C12R 1/89

(54) **METHOD FOR PRODUCING BIODIESEL BY TWO-STAGE CULTURE OF CHLORELLA FROM AUTOTROPHY TO HETEROTROPHY**
VERFAHREN ZUR HERSTELLUNG VON BIODIESEL MITTELS ZWEISTUFIGER CHLORELLA-KULTUR VON AUTOTROPHIE ZU HETEROTROPHIE
PROCÉDÉ DE PRODUCTION DE BIODIESEL PAR CULTURE EN DEUX ÉTAPES DE CHLORELLA DE L'AUTOTROPHIE VERS L'HÉTÉROTROPHIE

(30) Priority: 27.05.2008 CN 200810112998
(43) Date of publication of application: 09.03.2011
(73) Proprietor: Tsinghua University, Beijing 100084 (CN)
(72) Inventor: WU, Qing-Yu, Beijing 100084 (CN); XIONG, Wei, Beijing 100084 (CN)
(74) Representative: Michalski Hüttermann & Partner Patentanwälte mbB
(86) International application number: PCT/CN2008/072287
(87) International publication number: WO 2009/143680

(56) References cited:
- WO-A1-2008/083352
- WO-A1-2008/083352
- CN-A- 1 699 516
- CN-A- 1 837 352
- CN-A- 1 837 352
- US-A1- 2008 160 593
- US-A1- 2008 160 593
- XU, H. ET AL.: "High quality biodiesel production from a microalga Chlorella protothecoides by heterotrophic growth in fermenters", JOURNAL OF BIOTECHNOLOGY, vol. 126, no. 4, December 2006 (2006-12), pages 499-507, XP024956582,
- MIAO, X. & WU, Q.: "Biodiesel production from heterotrophic microalgal oil", BIORESOURCE TECHNOLOGY, vol. 97, no. 6, 4 June 2005 (2005-06-04), pages 841-846, XP025105819,
- WU, Q. ET AL.: "New Discoveries in Study on Hydrocarbons From Thermal Degradation of Heterotrophically Yellowing Algae", SCIENCE IN CHINA. SERIES B: CHEMISTRY, vol. 37, no. 3, March 1994 (1994-03), pages 326-335, XP008147361,
- WU, Q. ET AL.: "A COMPARATIVE STUDY OF GASES GENERATED FROM SIMULANT THERMAL DEGRADATION OF AUTOTROPHIC AND HETEROTROPHIC Chlorella", ZIRAN KEXUE JINZHAN = PROGRESS IN NATURAL SCIENCE, vol. 2, no. 4, August 1992 (1992-08), pages 311-318, XP008147393,
- LI, X. ET AL: "Large-Scale Biodiesel Production From Microalga Chlorella protothecoides Through Heterotropic Cultivation in Bioreactors", BIOTECHNOLOGY AND BIOENGINEERING, vol. 98, no. 4, 1 November 2007 (2007-11-01), pages 764-771, XP002583837,
- XIONG, W. ET AL.: "Double CO2 fixation in photosynthesis-fermentation model enhances algal lipid synthesis for biodiesel production", BIORESOURCE TECHNOLOGY, vol. 101, no. 7, 5 December 2009 (2009-12-05), pages 2287-2293, XP026822518,
- MIAO, X. L. ET AL.: 'Biodiesel production from heterotrophic microalgal oil.' BIORESOURCE TECHNOLOGY. vol. 97, no. 6, April 2006, pages 841 - 846, XP025105819
- WU, Q. Y. ET AL.: 'New discoveries in study on hydrocarbons from thermal degradation of heterotrophically yellowing algae.' SCIENCE IN CHINA (SERIES B). vol. 37, no. 3, March 1994, pages 326 - 335, XP008147361
- MIAO, X. L. ET AL.: 'Study on preparation of biodiesel from microalgal oil.' ACTA ENERGIAE SOLARIS SINICA. vol. 28, no. 2, February 2007, pages 219 - 222, XP008147362
- WU, Q. Y. ET AL.: 'A comparative study of gases generated from stimulant thermal degradation of autotrophic and heterotrophic Chlorella.' PROG NAT SCI (IN CHINESE) vol. 2, no. 4, 1992, pages 311 - 318, XP008147393

## Description

### Field of the Invention

This invention belongs to the field of renewable bio-energy, in particular relates to a new method for producing biodiesel through two-stage culture of Chlorella grown from autotrophic to heterotrophic cultivation. Specifically, the Chlorella cells are initially allowed to grow autotrophically using carbon dioxide under light conditions; then after the cells are concentrated, an organic carbon source (glucose) is added to the system to support heterotrophic growth of Chlorella, while lipids are significantly accumulated in Chlorella, thereby providing ample and inexpensive raw material for large-scale production of biodiesel.

### Background of the Invention

Biodiesel refers to monoalkyl esters of long chain fatty acids obtained by esterification reaction of animal, plant or microorganism lipids. Due to its clean, renewable, and environmental friendly properties, biodiesel industry has attracted increasing attention and rapidly developed in large energy-consuming countries. Currently, the "first generation" of biodiesel produced in Europe and America mostly utilizes economic crops (e.g. soybean, rapeseed, palm, etc.) as main raw material. Such bioenergy production depends on traditional agriculture, resulting in not only low productivity but also a competition with grain crops for natural resources (e.g. land, water, fertilizer, etc.), and thus the increasing demand for raw material in biodiesel industry cannot be fulfilled. Therefore, in order to relieve the pressure on agriculture caused by bioenergy industry, many international energy enterprises and manufacturing industries have begun to direct their efforts towards the use of a non-food material, microalgae, to develop so-called "second generation" of biofuels. (Peer MS, Skye RT, Evan S, et al.2008. Second Generation Biofuels: High-Efficiency Microalgae for Biodiesel Production. Bioenergy Research 1(1):20-43).

Microalgae are a group of photosynthetic microorganisms. They are capable of absorbing CO₂ from the atmosphere to synthesize lipids. After collected and processed, they can be refined to form biodiesel as automobile fuel, and even as jet fuel for aircraft after further refined. Due to the remarkable advantages of microalgae fuels (first they can grow in salty or waste water, and do not compete for agricultural land and fresh water; secondly, they have amazing productivity potential and can reproduce approximately 40 times faster than higher plants), they have great potential in dealing with global warming and satisfying the demand for non-polluting fuels. However, there are still many problems to be solved in biodiesel production using microalgae, so corresponding processes cannot yet fulfill requirements for commercialization.

At present, there are mainly two approaches for the large-scale cultivation of microalgae: autotrophic cultivation and heterotrophic fermentation, each of which has advantages and shortcomings in biodiesel production. Autotrophic cultivation is an environmental friendly way in which carbon dioxide, the main greenhouse gas, is fixed and oxygen is released. However, due to mutual shading among microalgae cells, light utilization is often limited. The shading effect becomes more obvious with the increase of cell concentration, and significantly affects the growth of cells and the synthesis of lipids, so it is difficult to obtain high-cell-density of oleaginous microalgae in photo-bioreactors. As for heterotrophic cultivation, the growth of cells depends mainly on the cells' absorption of organic carbon sources. Since it is not limited by light, a high density fermentation and efficient lipid synthesis could be easily realized through flow addition of organic carbon. In our group, a freshwater green algae *Chlorella protothecoides* that can grow both autotrophically and heterotrophically was first used in our research for biodiesel production by heterotrophic microalgae (See references: "Miao XL, Wu QY. 2006. Biodiesel production from heterotrophic microalgal oil. Bioresour Technol 97:841-846. ", "Xu H, Miao XL., Wu QY. 2006. High quality biodiesel production from microalga Chlorella protothecoides by heterotrophic growth in fermenters. J Biotechnol 126: 499-507. ", " Li XF, Xu H, Wu QY, 2007.Large-scale biodiesel production from microalga Chlorella protothecoids through heterotrophic cultivation in bioreactors. Biotechnology and Bioengineering. 98(4): 764-771.", "Xiong W,Li XF, Xiang JY,Wu QY. 2008 High-density fermentation of microalga Chlorella protothecoides in bioreactor for microbio-diesel production. Appl Microbiol Biotechnol 78(1):29-36"). Currently, through the establishment of an appropriate feed flow addition strategy, the density of heterotrophic *Chlorella* can reach up to 100 g·L⁻¹ with lipid content exceeding 50%. Heterotrophic cultivation has such advantages as high growth rate, short cultivation cycles and easy-to-control cultivation process, but the major problem of the heterotrophic cultivation of microalgae lies in the fact that this method depends on organic carbon (such as glucose, starch, etc) as raw material, which increases the cost of production.

Considering the advantages and shortcomings of autotrophic cultivation and heterotrophic fermentation, we have provided a process of manufacturing biodiesel by first autotrophical and then heterotrophical cultivation as follow: first allowing Chlorella to utilize inorganic carbon source (CO₂), and accumulating biomass under photoautotrophic conditions; then concentrating the cells; and supplementing them with organic carbon source (glucose), so as to allow Chlorella cells to accumulate lipids in great amount. Due to the direct utilization of light energy in the entire process, the efficiency of energy utilization is increased, the amount of organic carbon source used is decreased, and large amount of greenhouse gases is absorbed, making the process friendly to the environment. In another aspect, the cell concentrating technique is adopted in this process to avoid the problem of limited light in high concentration autotrophic cultivation. Subsequently, introduction of fermentation technique allows rapid lipid accumulation during heterotrophic growth of microalgae, which plays a key role in increasing the yield of biodiesel and in controlling costs.

### Content of the invention

The purpose of this invention is to provide a method of producing biodiesel through autotrophic cultivation followed by heterotrophic cultivation of Chlorella. Based on current research findings and patented technology for producing biodiesel through high concentration fermentation of heterotrophic Chlorella, we introduced procedures such as cultivating photoautotrophically, concentrating cells and preventing fermentation contamination and so on, so as to fulfill the requirements for large-scale industrialization of microalgae biodiesel. The key of this invention lies in the design and establishment of techniques and processes of autotrophic cultivation followed by heterotrophic cultivation of Chlorella, making Chlorella cells to rapidly grow and accumulate biomass under low concentration photoautotrophic conditions, and then synthesizing lipids under high concentration heterotrophic conditions after concentrating.

The invention concerns a method of producing biodiesel through a two-step autotrophic to heterotrophic cultivation of Chlorella, characterized in that the microalgae cells with lipid content higher than 50% obtained by firstly autotrophic cultivation and then heterotrophic cultivation are used as raw material for producing biodiesel; the method comprising the following steps: (1) autotrophically cultivating Chlorella; (2) concentrating cells; (3) heterotrophically fermenting; (4) collecting and drying algal cells; (5) extracting lipids from the dried algal cells; and (6) producing biodiesel by esterification reaction.

The technical processes, methods, and steps involved in the content of this invention are described in detail below:
(1) Photosynthetic autotrophic cultivation of Chlorella
   Freshwater green alga *Chlorella protothecoides* is inoculated into photo-bioreaction apparatus and allowed to grow with light in autotrophic medium; the photo-bioreaction apparatuses include aerated flask, photo-bioreactor, and open pond. The specific cultivation conditions are set as follow: temperature is controlled in the range of 20-45°C, the optimal temperature being 30°C; initial concentration of glycine is from 1 to 15 g.L⁻¹, with the preference of 5 g.L⁻¹; pH is controlled from 5 to 9, with the preference of 6.5; inflow of air or a mixture of air and CO₂ at a rate of 50-300 L/h, with the preference of 80-120L/h. Light intensity at 25-200µmol.m⁻²s⁻¹ is used during the cultivation process. The total cultivation time depends on the cell growth, and is generally from 50 to 400 hours, with the preference of from 120 to 200 hours.
(2) Concentrating of cells
   Cells are concentrated using high-speed centrifugation, flocculation or filtration technologies. For example, using high-speed centrifugation under sterile conditions, the centrifugal force is set as 2000-8000 g, and the centrifugation is performed at 4°C for 2-5 min. The supernatant is discarded after centrifugation, and the autotrophic cell pellet is retained.
(3)Heterotrophic fermentation
   In the process of converting Chlorella from autotrophic to heterotrophic growth, both the growth characteristics and the biochemical composition of the cells undergo obvious changes: the chlorophyll content decreases, the photosynthetic lamellae structure disappears, and the cell density decreases. By adding organic carbon, it is possible to direct most of the metabolic flow towards lipid synthesis pathway. For this purpose, the processes for high-cell-density heterotrophic cultivation and synthesis of lipids are set as follow:
   The collected autotrophically grown cells are re-suspended in sterile medium containing organic carbon source, making the initial density of the cells between 5 and 100g.L⁻¹, wherein the organic carbon source is selected from the group consisting of glucose, fructose, hydrolysates of corn, cassava, or wheat starch, and sorghum juice etc., and the initial reducing sugar concentration is between 0.01 and 100 g.L⁻¹, preferably 23g.L⁻¹.

   Heterotrophic fermentation can be performed in fermentation apparatuses of different volumes. Before fermentation, the electrodes of temperature, pH, Dissolved Oxygen (DO) and deforming electrode should be installed, and heterotrophic medium is added and autoclaved. The fermentation conditions are set as follow. Firstly, the pressure in the vessel, the air flow rate and stirring speed are adjusted to make the initial oxygen saturation reach 100%; the temperature is set between 15 and 45°C, preferably 29°C; the rate of air flow is between 100 and 300 L/h; and acid or base solution (KOH, H₂SO₄ etc) is added to maintain the pH of the fermentation system in the range of 5.0-9.0. During the fermentation process, the stirring speed is controlled to maintain the oxygen saturation above 10%. When carbon source runs out and the DO level suddenly rises, organic carbon source is fed to keep the concentration of the reducing sugar in the broth in the range of 1-40g.L⁻¹. Samples are taken regularly to determine the cell density and the concentration of the remaining reducing sugar in the medium to monitor carbon source consumption and cell growth. Nile Red staining and fluorescence assay are used to detect the change of neutral lipid in the cells. The fermentation should be terminated when the cell density exceeds 100g.L⁻¹ and the lipid content reaches more than 50% of the cells' dry weight. The total fermentation time is between 50 and 300 hours.
   To prevent possible contamination by undesired microorganisms during autotrophic cultivation and cell concentrating processes, antibacterial agents which inhibit microorganisms' growth are added. The antibacterial agents are chloramphenicol or sodium monofluoroacetate (MFA). Chloramphenicol is used at a final concentration between 0.002 and 0.2 g.L⁻¹, preferably 0.01 g.L⁻¹, or sodium monoflouroacetate (MFA) at a final concentration between 0.1 and 100 mM, preferably 2mM.
(4) Harvesting and drying algae cells
   Solid-liquid phase separation technology is employed to harvest algae cells from the fermentation broth. This process includes precipitation, filtration, centrifugation and drying. The algae cells obtained by separation are stored as dry powder, the drying methods including freeze-drying, spray drying, etc.
(5) Extraction of lipids from dried algae cells
   The method of extracting lipids from the dried algae cells comprises high pressure solvent extraction or Soxhlet extraction. When using Soxhlet extraction method, hexane is utilzed as the standard extraction solvent. Washing repeatedly with hexane, the lipids are separated from the algae powder until no lipids left in cells. Distillation is finally performed to remove the solvent.
(6) Esterification to generate biodiesel
   Esterification is carried out to make biodiesel from the lipids extracted from algal cells. The conversion from fatty acids to esters of fatty acids includes, but not limited to, catalysis with strong acid (e.g. concentrated sulfuric acid) or with lipase etc. The fatty acid methyl esters resulting from this catalysis are the main component of biodiesel.

The above discussed autotrophic cultivation medium consists of the following: KH₂PO₄: 0.2∼0.7 g.L⁻¹, K₂HPO₄: 0.1 ∼ 0.4 g.L^{- 1}, MgSO₄·7H₂O: 0.1 ∼ 0.4 g.L⁻¹, FeSO₄·7H₂O: 0.1 ∼ 3 mg.L⁻¹, glycine: 0.1 ∼ 15g.L⁻¹, vitamin B1: 0.001 ∼ 0.1mg.L⁻¹, A5 trace element fluid: 0.1 ∼ 5ml.L⁻¹; wherein the A5 trace element fluid, wich is known from CN 1 837 352 A,consists of: H₃BO₃: 2.86g.L , Na₂MoO₄·2H₂O: 0.039 g.L , ZnSO₄·7H₂O: 0.222 g.L , MnCl₂·4H₂O: 1.81 g.L , and CuSO₄·5H₂O: 0.074 g.L⁻¹.

The composition of heterotrophic cultivation medium is the same as the above, except for the absence of glycine and the addition of organic carbon source to maintain the initial reducing sugar concentration from 0.01 to 100 g.L⁻¹. During heterotrophic cultivation, organic carbon source is continuously added to maintain the concentration of reducing sugar in the range of 1-30 g.L⁻¹.

The benefits of the present invention lie in the fact that this invention adopts the process of first autotrophic cultivation and then heterotrophic cultivation, and respectively optimizing and combining the algal cell growth and fat synthesis, so the employed cell concentrating technique can effectively avoid the limited light problem in high-density autotrophic cultivation. This invention not only ensures higher light energy conversion efficiency, but also generates higher amount of fat. The finally obtained algae cell density (unit yield) reaches up to 108 g/L (dry weight), and the cell lipid content is more than 50%. Production capacity is also greatly improved over the traditional autotrophic cultivation, while glucose consumption and carbon dioxide emissions are significantly lower than that in heterotrophic cultivation. This technology which effectively controls the raw material costs of biodiesel and satisfies the requirements for industrial production of biodiesel by microalgae, is an economical and highly efficient novel approach to produce raw material lipids of biodiesel.

### The brief description of Figures

Fig. 1 Operational processes and flowchart of the production of biodiesel using Chlorella raised by autotrophic to heterotrophic two-stage cultivation.
Fig. 2 The autotrophic growth curve of Chlorella cells in a photo-bioreactor.
Fig. 3 The Growth curve and the glucose consumption curve of algae cells in a 5L bioreactor after conversion to heterotrophic cultivation. ((◆) glucose, (□)dry cell weight)
Fig. 4 The changes in fluorescence intensity of Nile Red stained cells measured by fluorescent spectrometry (Fig. 4B shows the staining results of autotrophic Chlorella, and Fig. 4A shows the staining results of Chlorella after conversion to heterotrophic cultivation. The emission peak of chlorophyll is at 680nm, and the emission peak of Nile Red is at 570 nm)

### Mode of carrying out the invention

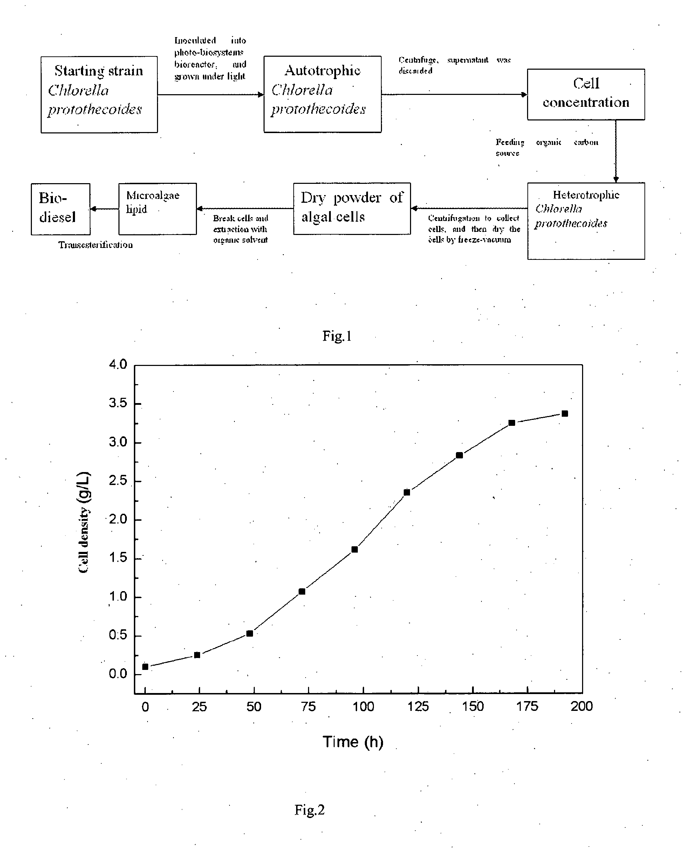

This invention provides a method of producing biodiesel using two-stage cultivation of Chlorella from autotrophic to heterotrophic stage. Based on current research findings and patented technologies for producing biodiesel through heterotrophic fermentation of microalgae, this invention further meets the requirements of large-scale industrial production of biodiesel using microalgae through cultivating photoautotrophic algae, concentrating cells and preventing undesired bacterial contamination etc. The technology, method, the following process steps as shown in Figure 1 and the invention are further illustrated by the provided examples.

### Examples

*Chlorella protothecoides,* an oleaginous microalgae strain was purchased from University of Texas Culture Collection of Algae in the United States. Since 1990, this strain has been cultured in the Algal Bio-Energy Lab of the Department of Biological Science and Biotechnology at Tsinghua University for bio-energy research. The autotrophic medium consisted of : KH₂PO₄: 0.6 g.L⁻¹, K₂HPO₄: 0.4 g.L⁻¹, MgSO₄·7H₂O: 0.4 g.L⁻¹, FeSO₄·7H₂O: 1 mg.L⁻¹, glycine : 3g.L⁻¹, vitamin B1: 0.03 mg.L⁻¹, and A5 trace element solution 1.5ml.L⁻¹, wherein the A5 trace element solution consisted of H₃BO₃: 2.86g.L , Na₂MoO₄·2H₂O: 0.039 g.L⁻¹, ZnSO₄·7H₂O: 0.222 g.L⁻¹, MnCl₂·4H₂O: 1.81 g.L⁻¹, and CuSO₄·5H₂O: 0.074 g.L⁻¹;

As described in step (1), a single colony of Chlorella grown on solid medium was inoculated to a 1L aerated flask to make an initial cell density of 0.1 g.L⁻¹. The flask was placed in a light chamber and incubated at 25°C, and air was circulated at a flow rate of 100 L/h. pH of the culture was controlled in the range of 5-9. During the autotrophic cultivation, the effects of different cultivation conditions, such as nitrogen sources, light intensity etc. on the growth of Chlorella cells were investigated. The optimizing process of the growth conditions was as follows: 1, 3, 5, 7, or 9 g.L⁻¹ of glycine was respectively used in the medium, and the cultivation was carried out under light intensities of 25, 50, 100 or 200 µmol.m⁻² s⁻¹. The optimization results showed that, under the above temperature and air circulation conditions, the cells grew best with glycine at 5g.L⁻¹ and illumination intensity of 100µmol.m⁻²s⁻¹ (as shown in Fig. 2).

When the cells entered the ending stage of the log phase, the air bubbling was stopped and the culture was kept still for 12 hours to allow the cells to settle down. The supernatant was discarded and the remaining culture medium was further removed by centrifugation at 3000g for 2 min. The process was performed under sterile condition.

A 5L fermentor (MINIFORS, Switzerland) was prepared, with the electrodes of temperature, pH, DO (adjusted) and the deforming electrode adjusted, then heterotrophic medium was added, and sterilized at 121°C for 30 minutes. The composition of the heterotrophic medium was as follow: KH₂PO₄: 0.6 g.L⁻¹, K₂HPO₄: 0.4 g.L⁻¹, MgSO₄·7H₂O: 0.4 g.L⁻¹, FeSO₄·7H₂O: 0.5 mg.L⁻¹, vitamin B1: 0.08 mg.L⁻¹, A₅ trace element liquid 1.5 ml.L⁻¹, and glucose 23g.L⁻¹. Sodium monoflouroacetate (MFA) was added to a final concentration of 1 mM in order to prevent microbial contamination. The collected autotrophic Chlorella cells were resuspended and transferred to the fermentor, making the initial cell density of 20 g.L⁻¹.

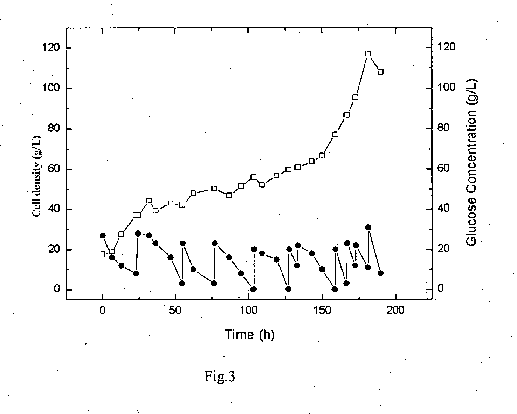

The conditions for heterotrophic cultivation were set as follow: temperature was maintained at 29°C, air flow rate was set as 150 L/h, pH was adjusted to 6.2±0.2 through addition of acid or base (e.g. KOH, H₂SO₄, etc.). The dissolved oxygen was monitored on line. Once the dissolved oxygen was lower than 10% of the saturation, the stirring rate was then gradually increased so as to maintain the saturation of the dissolved oxygen in the fermentor above 10%. When carbon source was depleted and the dissolved oxygen (DO) level steeply increased, glucose was added to maintain the concentration of glucose as reducing sugar in the range of 1-30 g.L⁻¹. A high-density cultivation strategy was set up through fed-batch and process control in a 5L fenmentor: the changes in dissolved oxygen, glucose, and cell density in the fermentor were monitored, while the neutral lipid content in the algae cells was also measured using Nile Red mediated fluorescence technique (c.f. specific procedures described in "Danielle E, David J, Barry R, et al. 2007.Fluorescent measurement of microalgal neutral lipids. Journal of Microbiological Methods, 68 (3): 639-642."). The cell density during the fermentation process was estimated by periodic measurements of optical density (OD₅₄₀ₙₘ). The linear relationship between OD₅₄₀ₙₘ and the dry weight of the cells may be expressed by the following equation: y = 0.4155x, (R² = 0.9933, P < 0.05), where y represents cell density (g.L⁻¹), and x represents the optical density at 540 nm. After fermentation, cells were harvested from the fermentation broth by centrifugation, and then dried in vacuum. The fermentation broth was centrifuged at 8000 g for 2 min, and the pellet was collected and then dried in vacuum. After weighing, 108 g.L⁻¹ of dry algae powder in total was obtained (see Fig. 3).

Using Soxhlet extractor, a semiautomatic solvent extraction device with hexane as the standard solvent, lipids were extracted from the algae powder. The cells were repeatedly washed with hexane in the Soxhlet extractor, until no lipids were left in the cells. The molecular weights of the lipids from heterotrophically cultivated algae were calculated as follow:
Molecular weight (M) = 561×1000×3/(SV-AV), where SV represents the saponification value of microalgae lipids, and AV (acid value) represents the acid value of the microalgae lipids, and the resulting molecular mass value calculated is a dimensionless number.

After rotary evaporation with solvent decompression, the lipids obtained by Soxhlet extraction were dried and then weighed. The net weight of lipids was divided by the dry weight of cells, and the lipid content in the cell dry weight was 52% (w/w). The total consumption of glucose throughout the cultivation process was 280 g.L⁻¹, and lipid yield was 56.16g.L⁻¹(i.e. 108 g.L⁻¹×52%), indicating a conversion rate of 20.06%. According to our previous experimental data, when using high density heterotrophic fermentation, the sugar-to-lipid conversion rates were approximately from 10 to 17%. This indicates that under equivalent conditions, the two-step cultivation process can effectively lower the consumption of organic carbon source.

Esterification was performed through concentrated sulfuric acid catalysis. A certain amount of algae lipids were placed in a flask, and an equal amount of concentrated sulfuric acid was added as catalyst. Methanol solvent was also added so that the molar ratio of methanol to lipid was 30:1. The reaction was carried out in an incubator at 55°C with a rotary rate of 160 revolutions per minute for 48 hours. After the reaction was completed, the reaction mixture formed two layers. The biodiesel contained in the upper layer were isolated and washed with warm water at 30 °C. Then rotary evaporation was carried out to remove the solvent and the pure biodiesel was obtained.

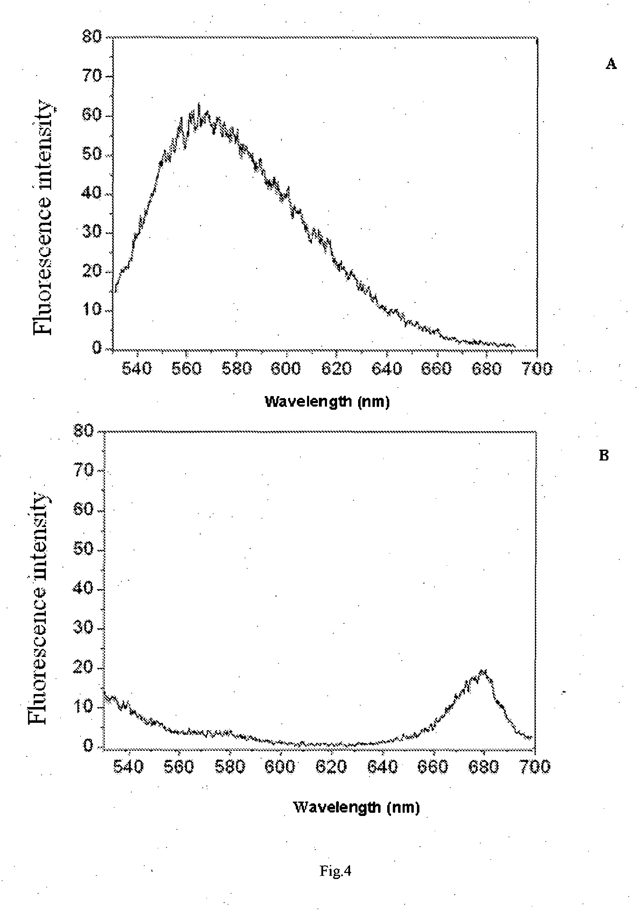

Through chromatography - mass spectrometry, the content of tri-, di-, and monoglycerides, methanol, and glycerol etc. in the system can be analyzed, based on which the conversion rate of lipid to fatty acid methyl ester can be calculated. DSQ GC (Thermo, USA , VARIAN VF-5ms Capillary 30M*0.25MM) gas chromatography - mass spectrometry was employed for analysis. The flow rate was set at 10 ml min⁻¹. The procedure for raising temperature was as follow: the temperature was first increased to 70 °C and maintained for 2 min; then was increased to 300 °C at the rate of 10 °C min⁻¹, and maintained for 20 min. Temperature of inject entrance was set at 250°C with flow ratio of 30:1. During the 48 hours' esterification reaction, more than 90% of the algae lipids could be converted to fatty acid methyl esters (biodiesel), and the main components of biodiesel were consistent with those of the algal oil derived from heterotrophic fermentation (see Fig. 4).

## Claims

1. A method of producing biodiesel through a two-step autotrophic to heterotrophic cultivation of Chlorella, **characterized in that** the microalgae cells with lipid content higher than 50% obtained by firstly autotrophic cultivation and then heterotrophic cultivation are used as raw material for producing biodiesel; the method comprising the following steps: (1) autotrophically cultivating Chlorella; (2) concentrating cells; (3) heterotrophically fermenting; (4) collecting and drying algal cells; (5) extracting lipids from the dried algal cells; and (6) producing biodiesel by esterification reaction.

2. The method of producing biodiesel through a two-step autotrophic to heterotrophic cultivation of Chlorella according to claim 1, **characterized in that** the Chlorella is freshwater green algae *Chlorella protothecoides.*

3. The method of producing biodiesel through a two-step autotrophic to heterotrophic cultivation of Chlorella according to claim 1, **characterized in that** the autotrophically cultivating Chlorella of said step (1) comprises: inoculating the freshwater green algae *Chlorella protothecoides* into a flask, cultivation pond or photo-bioreactor for incubating under light, with a temperature in the range of 20-45°C; glycine with an initial concentration of 1 to 15 g.L⁻¹; a pH between 5 and 9; an air circulation rate between 50 and 300 L/h; and 25-200µmol.m⁻²s⁻¹ of light during the cultivation process, resulting in an optimal cell growth.

4. The method of producing biodiesel through a two-step autotrophic to heterotrophic cultivation of Chlorella according to claim 1, **characterized in that** the method for concentrating cells in said step (2) is high-speed centrifugation, flocculation, or filtration.

5. The method of producing biodiesel through a two-step autotrophic to heterotrophic cultivation of Chlorella according to claim 1, **characterized in that** in heterotrophic fermenting of said step (3), the initial cell density is controlled between 5 and 100 g.L⁻¹, the temperature is from 15 to 45°C, air flow rate is from 100 to 300L/h, and the pH is controlled in the range of 5.0 to 9.0 by adding acid or base solution; the reducing sugar concentration in the broth is maintained in the range of 1 to 40 g.L⁻¹ by supplementing organic carbon source, the stirring rate is controlled to maintain the oxygen saturation in the broth above 10%; chloramphenicol or sodium monofluoroacetate is added to prevent possible microbial contamination during autotrophic cultivation and cell concentration processes, the final concentration of chloramphenicol is between 0.002 and 0.2 g.L⁻¹, and the final concentration of sodium monofluoroacetate (MFA) is between 0.1 and 100mM.

6. The method of producing biodiesel through a two-step autotrophic to heterotrophic cultivation of Chlorella according to claim 1, **characterized in that**, the composition of said autotrophic medium is: KH₂PO₄: 0.2 ∼0.7 g.L⁻¹, K₂HPO₄: 0.1∼0.4 g.L⁻¹, MgSO₄·7H₂O: 0.1∼0.4 g.L⁻¹, FeSO₄·7H₂O: 0.1∼3 mg.L⁻¹, glycine: 0.1-15 g.L⁻¹, vitamin B1: 0.001∼0.1mg.L⁻¹, and A5 trace element liquid 0.1∼5ml.L⁻¹, wherein the A5 trace element liquid consists of H₃BO₃: 2.86g.L⁻¹, Na₂MoO₄·2H₂O: 0.039 g.L⁻¹, ZnSO₄·7H₂O: 0.222 g.L MnCl₂·4H₂O: 1.81 g.L⁻¹, and CuSO₄·5H₂O: 0.074 g.L⁻¹; the composition of the heterotrophic medium is the same as above, except for the absence of glycine and the addition of organic carbon source to make the initial reducing sugar concentration of between 0.01 and 100 g.L⁻¹; during heterotrophic cultivation, organic carbon source is continuously added to maintain the concentration of reducing sugar in the range of 1-30 g.L⁻¹.

7. The method of producing biodiesel through a two-step autotrophic to heterotrophic cultivation of Chlorella according to claim 1, **characterized in that**, an organic carbon source is added into the heterotrophic medium as reducing sugar which includes glucose or other monosaccharides, disaccharides or polysaccharides; the organic carbon source comprising glucose, fructose, corn starch hydrolysate, cassava starch hydrolysate, wheat starch hydro lysate, or sorghum juice.

## Patentansprüche

1. Verfahren zur Produktion von Biodiesel durch eine zweistufige autotrophe bis heterotrophe Anzucht von Chlorella, **dadurch gekennzeichnet, dass** die durch zunächst autotrophe Anzucht und dann heterotrophe Anzucht erhaltenen Mikroalgenzellen mit einem höheren Lipidgehalt als 50% als Rohstoff für die Produktion von Biodiesel verwendet werden; wobei das Verfahren die folgenden Schritte umfasst: (1) autotrophe Anzucht von Chlorella; (2) Konzentrieren der Zellen; (3) heterotrophe Fermentation; (4) Sammeln und Trocknen von Algenzellen; (5) Extrahieren von Lipiden aus den getrockneten Algenzellen; und (6) Produktion von Biodiesel durch Veresterungsreaktion.

2. Verfahren zur Produktion von Biodiesel durch eine zweistufige autotrophe bis heterotrophe Anzucht von Chlorella nach Anspruch 1, **dadurch gekennzeichnet, dass** es sich bei der Chlorella um die Süßwasser-Grünalge *Chlorella protothecoides* handelt.

3. Verfahren zur Produktion von Biodiesel durch eine zweistufige autotrophe bis heterotrophe Anzucht von Chlorella nach Anspruch 1, **dadurch gekennzeichnet, dass** die autotrophe Anzucht von Chlorella aus Schritt (1) umfasst: Animpfen der Süßwasser-Grünalge *Chlorella protothecoides* in einen Kolben, ein Anzuchtbecken oder einen Photobioreaktor zur Inkubation unter Licht mit einer Temperatur im Bereich von 20-45°C; Glycin mit einer Anfangskonzentration von 1 bis 15 g.l⁻¹; einem pH zwischen 5 und 9; einer Luftzirkulationsrate zwischen 50 und 300 l/Std.; und 25-200 µmol.m⁻²s⁻¹ Licht während des Anzuchtprozesses, was zu einem optimalen Zellwachstum führt.

4. Verfahren zur Produktion von Biodiesel durch eine zweistufige autotrophe bis heterotrophe Anzucht von Chlorella nach Anspruch 1, **dadurch gekennzeichnet, dass** das Verfahren zum Konzentrieren von Zellen in Schritt (2) Hochgeschwindigkeitszentrifugation, Ausflockung oder Filtration ist.

5. Verfahren zur Produktion von Biodiesel durch eine zweistufige autotrophe bis heterotrophe Anzucht von Chlorella nach Anspruch 1, **dadurch gekennzeichnet, dass** bei der heterotrophen Fermentation in Schritt (3) die Anfangszelldichte zwischen 5 und 100 g.l⁻¹ gesteuert wird, die Temperatur 15 bis 45°C beträgt, die Luftströmungsrate 100 bis 300 l/Std. beträgt und der pH-Wert durch Zugabe von Säure- oder Basenlösung im Bereich von 5,0 bis 9,0 eingestellt wird; die reduzierende Zuckerkonzentration in der Brühe im Bereich von 1 bis 40 g.l^{-l} durch Ergänzung der organischen Kohlenstoffquelle gehalten wird, die Rührgeschwindigkeit derart gesteuert wird, dass die Sauerstoffsättigung in der Brühe über 10% gehalten wird; Chloramphenicol oder Natriummonofluoracetat zugegeben wird, um eine mögliche mikrobielle Kontamination während der autotrophen Anzucht- und Zellkonzentrationsprozesse zu verhindern, die Endkonzentration von Chloramphenicol zwischen 0,002 und 0,2 g.l^{-l} liegt und die Endkonzentration von Natriummonofluoracetat (MFA) zwischen 0,1 und 100 mM liegt.

6. Verfahren zur Produktion von Biodiesel durch eine zweistufige autotrophe bis heterotrophe Anzucht von Chlorella nach Anspruch 1, **dadurch gekennzeichnet, dass** die Zusammensetzung des autotrophen Mediums ist: KH₂PO₄: 0,2∼0,7 g.l⁻¹, K₂HPO₄: 0,1∼0,4 g.l⁻¹, MgSO₄ · 7H₂O: 0,1∼0,4 g.l⁻¹, FeSO₄ · 7H₂O: 0,1∼3 mg.l⁻¹, Glycin: 0,1∼15 g.l⁻¹, Vitamin B1: 0,001∼0,1 mg.l⁻¹ und A5 Spurenelementflüssigkeit 0,1∼5 ml.l⁻¹, wobei die A5-Spurenelementflüssigkeit aus H₃BO₃: 2,86 g.l⁻¹, Na₂MoO₄ · 2H₂O: 0,039 g.l⁻¹, ZnSO₄ · 7H₂O: 0,222 g.l⁻¹, MnCl₂ · 4H₂O: 1,81 g.l^{-l} und CuSO₄ · 5H₂O: 0,074 g.l^{-l} besteht; wobei die Zusammensetzung des heterotrophen Mediums die gleiche ist wie oben, außer der Abwesenheit von Glycin und der Zugabe der organischen Kohlenstoffquelle, um die anfängliche reduzierende Zuckerkonzentration zwischen 0,01 und 100 g.l⁻¹ einzustellen; während der heterotrophen Anzucht wird die organische Kohlenstoffquelle kontinuierlich zugesetzt, um die Konzentration des reduzierenden Zuckers im Bereich von 1-30 g.l^{-l} zu halten.

7. Verfahren zur Produktion von Biodiesel durch eine zweistufige autotrophe bis heterotrophe Anzucht von Chlorella nach Anspruch 1, **dadurch gekennzeichnet, dass** eine organische Kohlenstoffquelle dem heterotrophen Medium als reduzierender Zucker zugesetzt wird, der Glucose oder andere Monosaccharide, Disaccharide oder Polysaccharide umfasst; wobei die organische Kohlenstoffquelle Glucose, Fructose, Maisstärkehydrolysat, Cassavastärkehydrolysat, Weizenstärkehydrolysat oder Sorghum-Saft umfasst.

## Revendications

1. Méthode de production d'un biodiesel par l'intermédiaire d'une culture autotrophe à hétérotrophe à deux étapes de *Chlorella,* **caractérisée en ce que** les cellules de microalgues ayant une teneur en lipides supérieure à 50% obtenues d'abord par une culture autotrophe puis une culture hétérotrophe sont utilisées comme matière première pour la production de biodiesel ; la méthode comprenant les étapes suivantes : (1) la culture autotrophe de Chlorella ; (2) la concentration des cellules ; (3) la fermentation par voie hétérotrophe ; (4) la récupération et le séchage des cellules algales ; (5) l'extraction des lipides à partir des cellules algales séchées ; et (6) la production de biodiesel par une réaction d'estérification.

2. Méthode de production d'un biodiesel par l'intermédiaire d'une culture autotrophe à hétérotrophe à deux étapes de *Chlorella* selon la revendication 1, **caractérisée en ce que** *Chlorella* est l'algue verte d'eau douce *Chlorella protothecoides.*

3. Méthode de production d'un biodiesel par l'intermédiaire d'une culture autotrophe à hétérotrophe à deux étapes de *Chlorella* selon la revendication 1, **caractérisée en ce que** la culture par voie autotrophe de *Chlorella* de ladite étape (1) comprend : l'inoculation de l'algue verte d'eau douce *Chlorella protothecoides* dans un flacon, un étang de culture ou un photobioréacteur pour une incubation sous lumière, avec une température dans la plage de 20-45°C ; de la glycine selon une concentration initiale allant de 1 à 15 g.l⁻¹ ; un pH compris entre 5 et 9 ; un débit de circulation d'air compris entre 50 et 300 l/h ; et 25-200 µmol.m⁻²s⁻¹ de lumière lors du procédé de culture, conduisant à une croissance cellulaire optimale.

4. Méthode de production d'un biodiesel par l'intermédiaire d'une culture autotrophe à hétérotrophe à deux étapes de *Chlorella* selon la revendication 1, **caractérisée en ce que** la méthode de concentration des cellules dans ladite étape (2) est une centrifugation à haute vitesse, une floculation, ou une filtration.

5. Méthode de production d'un biodiesel par l'intermédiaire d'une culture autotrophe à hétérotrophe à deux étapes de *Chlorella* selon la revendication 1, **caractérisée en ce que**, dans la fermentation hétérotrophe de ladite étape (3), la densité cellulaire initiale est contrôlée entre 5 et 100 g.l⁻¹, la température va de 15 à 45°C, le débit d'air va de 100 à 300 l/h, et le pH est contrôlé dans la plage allant de 5,0 à 9,0 par l'addition d'une solution d'acide ou de base ; la concentration en sucres réducteurs dans le bouillon est maintenue dans la plage allant de 1 à 40 g.l^{-l} par une complémentation en source de carbone organique, la vitesse d'agitation est contrôlée afin de maintenir la saturation en oxygène dans le bouillon au-dessus de 10% ; on ajoute du chloramphénicol ou du monofluoroacétate de sodium afin d'empêcher une éventuelle contamination microbienne lors des procédés de culture autotrophe et de concentration cellulaire, la concentration finale en chloramphénicol est comprise entre 0,002 et 0,2 g.l⁻¹, et la concentration finale en monofluoroacétate de sodium (MFA) est comprise entre 0,1 et 100 mM.

6. Méthode de production d'un biodiesel par l'intermédiaire d'une culture autotrophe à hétérotrophe à deux étapes de *Chlorella* selon la revendication 1, **caractérisée en ce que** la composition dudit milieu autotrophe est : KH₂PO₄ : 0,2-0,7 g.l⁻¹, K₂HPO₄ : 0,1-0,4 g.l⁻¹, MgSO₄·7H₂O : 0,1-0,4 g.l⁻¹, FeSO₄·7H₂O : 0,1-3 mg.l⁻¹, glycine : 0,1-15 g.l⁻¹, vitamine B1 : 0,001-0,1 mg.l⁻¹, et oligo-éléments liquides A5 : 0,1-5 ml.l⁻¹, où les oligo-éléments liquides A5 sont constitués par H₃BO₃ : 2,86 g.l⁻¹, Na₂MoO₄·2H₂O : 0,039 g.l⁻¹, ZnSO₄·7H₂O : 0,222 g.l, MnCl₂·4H₂O : 1,81 g.l⁻¹, et CuSO₄·5H₂O : 0,074 g.l^{-l} ; la composition du milieu hétérotrophe est la même que ci-dessus, à l'exception de l'absence de glycine et de l'addition d'une source de carbone organique afin que la concentration en sucres réducteurs initiale soit comprise entre 0,01 à 100 g.l^{-l} ; lors de la culture hétérotrophe, la source de carbone organique est ajoutée en continu afin de maintenir la concentration en sucres réducteurs dans la plage de 1-30 g.l⁻¹.

7. Méthode de production d'un biodiesel par l'intermédiaire d'une culture autotrophe à hétérotrophe à deux étapes de *Chlorella* selon la revendication 1, **caractérisée en ce qu'**une source de carbone organique est ajoutée au milieu hétérotrophe sous forme de sucres réducteurs qui comportent le glucose et autres monosaccharides, disaccharides ou polysaccharides ; la source de carbone organique comprenant du glucose, du fructose, un hydrolysat d'amidon de maïs, un hydrolysat d'amidon de manioc, un hydrolysat d'amidon de blé, ou du jus de sorgho.
